# EUROPEAN PATENT SPECIFICATION

(11) **EP 3 347 078 B1**
(45) Date of publication and mention of the grant of the patent: **25.10.2023**
(21) Application number: 15770740.7
(22) Date of filing: 11.09.2015
(51) Int. Cl.: A61M 25/00

(54) **POLYMERIC CATHETER SHAFT WITH REINFORCEMENT**
POLYMERKATHETERSCHAFT MIT VERSTÄRKUNG
CORPS DE CATHÉTER EN POLYMÈRE, POURVU D'UN RENFORT

(43) Date of publication of application: 18.07.2018
(73) Proprietor: Covidien LP, Mansfield, MA 02048 (US)
(72) Inventor: LIM, Elaine, Mountain View, CA 94043 (US); YE, Ting, Tina, Mountain View, CA 94043 (US)
(74) Representative: Elsy, David
(86) International application number: PCT/US2015/049801
(87) International publication number: WO 2017/044131

(56) References cited:
- EP-A1- 0 661 072
- EP-A1- 2 174 685
- WO-A1-03/086519
- US-A1- 2006 089 618
- US-A1- 2011 238 041

## Description

### TECHNICAL FIELD

The present technology relates generally to catheters. More specifically, the invention relates to catheter shaft construction.

### BACKGROUND

A wide variety of medical devices have been developed for intravascular use. Catheters, for example, are commonly used to facilitate navigation through and/or treatment within the anatomy of a patient. To direct the distal portion of the catheter to the correct location in the vasculature, a physician must apply longitudinal forces, and sometimes rotational forces (i.e., torsional forces), from the proximal end of the catheter. For the catheter shaft to transmit these forces from the proximal end to the distal end, the catheter must be sufficiently rigid to be pushed through the blood vessel (a property commonly referred to as "pushability"), yet flexible enough to navigate through the often tortuous bends in the blood vessel. The catheter may also require sufficient torsional stiffness to transmit the applied torque (a property commonly referred to as "torqueability"). A need exists for catheter shafts that accomplish a balance between longitudinal rigidity, torsional stiffness, and flexibility.

U.S. Patent Application Publication No. 2011/0238041 A1 by Lim et al. describes a multi-layered catheter having various sections of different flexibility extending distally along the length of the catheter. European Patent Application Publication EP 2 174 685 A1 by Asahi Intecc Co Ltd. describes a catheter that includes a hollow coil whose outer and inner surfaces are coated with outer and inner resin layers. The catheter also includes a tapered tip and a braid embedded in the catheter body and the tip. International Publication No. WO 03/086519 A1 by Biosphere Medical describes a reinforced catheter that includes a helical strand that has a stiffness of about 30 % to about 100 % greater than the stiffness of other helical strands used for reinforcing the catheter. U.S. Patent Application Publication No. 006/089618 A1 by McFerran et al. describes a catheter having a preshaped tip configuration. The catheter may include features that transition the stiffness and flexibility of the catheter. European Patent Application Publication EP 0 661 072 A1 by Terumo Corp. describes a catheter that includes an intermediate layer including a double layer coil portion and a rigid layer.

### BRIEF DESCRIPTION OF THE DRAWINGS

Many aspects of the present technology can be better understood with reference to the following drawings. The components in the drawings are not necessarily to scale. Instead, emphasis is placed on illustrating clearly the principles of the present disclosure.
Figure 1A is a side view of a catheter in accordance with the present technology.
Figure 1B is a cross-sectional side view of a portion the catheter shaft shown in Figure 1A.
Figure 2 is a cross-sectional side view of a portion of an elongated catheter shaft configured in accordance with another embodiment of the present technology.
Figure 3 is a cross-sectional side view of a distal portion of an elongated catheter shaft configured in accordance with the present technology.
Figure 4 is a cross-sectional side view of a distal portion of an elongated catheter shaft configured in accordance with the present technology.
Figure 5 is a cross-sectional side view of a distal portion of an elongated catheter shaft configured in accordance with the present technology.
Figure 6 is a cross-sectional side view of a distal portion of an elongated shaft configured in accordance with the present technology.
Figure 7 is a cross-sectional side view of a distal portion of an elongated shaft configured in accordance with the present technology.
Figure 8 is a cross-sectional side view of a distal portion of an elongated shaft configured in accordance with the present technology.

### DETAILED DESCRIPTION

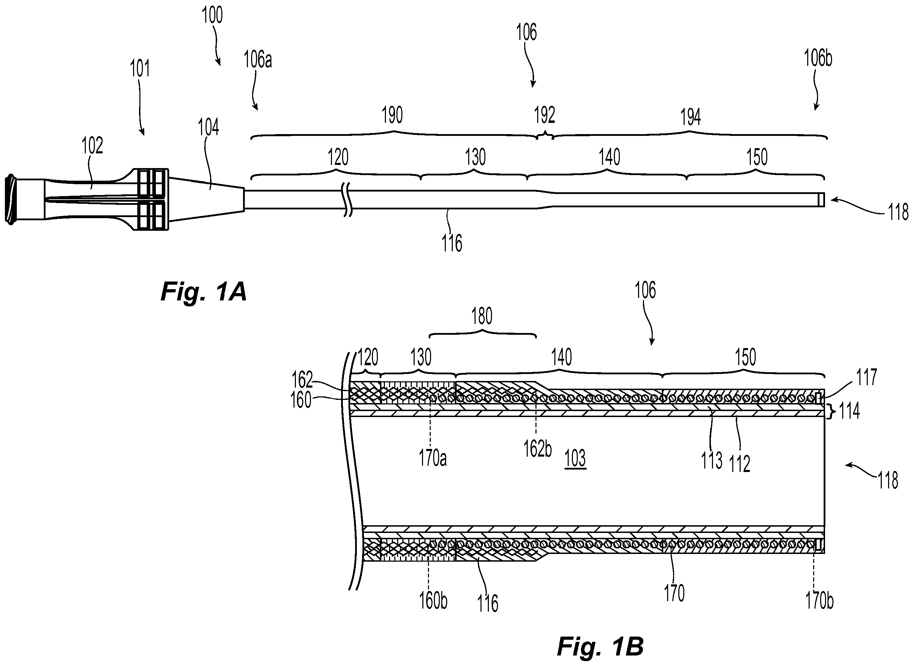

The present invention is directed to catheters as defined in the claims. Specific details of several embodiments of catheter devices, systems, and methods in accordance with the present technology are described below with reference to Figures 1A-8. With regard to the terms "distal" and "proximal" within this description, unless otherwise specified, the terms can reference a relative position of the portions of a catheter and/or an associated device with reference to an operator and/or a location in the vasculature. Also, the term "thickness" as used herein with respect to a particular material or layer refers to the perpendicular distance between the plane running through and generally parallel with the radially outermost surface of the particular material or layer and the plane running through and generally parallel with the radially innermost surface of the particular material or layer.

The methods herein do not form part of the claimed invention.

### I. Selected Embodiments of Catheter Shafts of the Present Technology

Figure 1A is a side view of a catheter 100 configured in accordance with an embodiment of the present technology, and Figure 1B is a cross-sectional side view of a portion of the catheter 100 shown in Figure 1A. Referring to Figures 1A-1B together, the catheter 100 includes a handle assembly 101 and an elongated shaft 106 having a proximal portion 106a coupled to the handle assembly 101 and a distal portion 106b. The handle assembly 101 includes a hub 102 configured to facilitate connection to other devices (e.g., a syringe, a Y-adapter, etc.) and a transition portion 104 configured to provide strain relief at the proximal portion 106a. In other embodiments, the handle assembly 101 can have other suitable configurations based on the desired functions and characteristics of the catheter 100.

The shaft 106 is a generally tubular member having an inner surface that defines a lumen 103 (Figure 1B) extending from the proximal portion 106a of the shaft 106 to an opening 118 at the distal terminus of the distal portion 106b. In some embodiments, the shaft 106 can include a radiopaque marker 117 (Figure 1B) surrounding the lumen 103 at or just proximal to the opening 118. The lumen 103 is configured to slidably receive and facilitate the passage therethrough of one or more medical devices, such as guidewires, balloon catheters, implants, intrasaccular occlusion devices (e.g., coils, expandable cages, expandable meshes, etc.), infusion devices, stents and/or stent-grafts, intravascular occlusion devices, clot retrievers, implantable heart valves, and other suitable medical devices and/or associated delivery systems. Additionally, the lumen 103 is configured to receive one or more fluids therethrough, such as radiopaque dye, saline, drugs, and the like.

The size of the lumen 103 can vary, depending on the desired characteristics of the catheter 100. For example, in some embodiments the shaft 106 can have an inner diameter (e.g., lumen diameter) between about 0.254 millimeters (about 0.01 inches) and about 1.27 millimeters (about 0.05 inches) (e.g., 0.4318 millimeters (0.017 inches), 1.1303 millimeters (0.0445 inches, etc.), and in some embodiments between about 0.508 millimeters (about 0.02 inches) and 1.143 millimeters (about 0.045 inches) (e.g., 0.5334 millimeters (0.021 inches), etc.). In a particular embodiment, the inner diameter is between about 0.635 millimeters (about 0.025 inches) and about 1.016 millimeters (about 0.04 inches) (e.g., 0.6858 millimeters (0.027 inches, 0.8128 millimeters (0.032 inches, etc.). Although the shaft 106 shown in Figure 1A has a generally round cross-sectional shape, it will be appreciated that the shaft 106 can include other cross-sectional shapes or combinations of shapes. For example, the cross-sectional shape of the shaft 106 can be oval, rectangular, square, triangular, polygonal, and/or any other suitable shape and/or combination of shapes.

The outer diameter of the shaft 106 can be the same or vary along its length. For example, in the embodiment shown in Figures 1A-1B, the shaft 106 has a first portion 190 with a first diameter, a tapered portion 192 with a diameter that decreases in a proximal to distal direction, and a second portion 194 with a second diameter less than the first diameter. The length of the tapered portion 192 can be between about 1 cm and about 5 cm. In some embodiments, the shaft 106 does not include a second portion 194 and the tapered portion 192 extends distally to the distal terminus of the shaft 106. In other embodiments, the shaft 106 has an outer diameter that is generally constant along its length. Moreover, the length and/or outside diameter of the shaft 106 is generally selected for the desired use of the catheter 100. For example, in those embodiments where the catheter 100 is configured as a guide catheter for enabling intravascular insertion and navigation, the outside diameter of the shaft 106 can be between about 1 millimeters (about 3 Fr) and about 3.333 millimeters (about 10 Fr). In those embodiments where the catheter 100 is configured as a microcatheter for use within small anatomies of the patient, the outside diameter of the shaft 106 can be between about 0.333 millimeters (about 1 Fr) and about 1 millimeters (about 3 Fr).

Many embodiments of the present technology are particularly useful in treating targets located in tortuous and narrow vessels, such as certain sites in the neurovascular system, the coronary vascular system, or the peripheral vascular system (e.g., the superficial femoral, popliteal, or renal arteries). Neurovascular target sites, such as sites in the brain, are often accessible only via a tortuous vascular path. Although some embodiments of the catheter 100 are described in terms of intravascular use, in other embodiments the catheter 100 may be suited for uses in the digestive system, soft tissues, and/or any other insertion into an organism for medical uses. For example, in some embodiments, the catheter 100 may be significantly shorter and used as an introducer sheath, while in other embodiments the catheter 100 may be adapted for other medical procedures.

In the embodiment shown in Figure 1B, the elongated shaft 106 includes an inner polymer structure 114 and an outer polymer structure 116 surrounding at least a portion of the inner polymer structure 114. The shaft 106 shown in Figure 1B also has an inner braid 160 embedded in the outer polymer structure 116, an outer braid 162 surrounding at least a portion of the inner braid 160, and a coil 170 wrapped around at least a portion of the inner polymer structure 114. Each of these subcomponents will now be described in greater detail.

Referring again to Figures 1A-1B together, the inner polymer structure 114 extends from the proximal portion 106a of the shaft 106 to a location within the distal portion 106b of the shaft 106. For example, in the embodiment shown in Figure 1B, the inner polymer structure 114 extends from the proximal portion 106a of the shaft 106 to the opening 118 at the distal terminus of the distal portion 106b (e.g., the entire length of the shaft 106 or substantially the entire length of the shaft 106). In other embodiments, the inner polymer structure 114 extends along only a portion of the length of the shaft 106 and/or has a proximal and/or a distal terminus that does not correspond to a proximal terminus and/or a distal terminus, respectively, of the shaft 106. The length of the inner polymer structure 114 can vary depending upon, for example, the length of the shaft 106 and the desired characteristics and functions of the catheter 100.

The inner polymer structure 114 can be made of any suitable polymer (and/or combination of multiples polymers) and by any suitable process. Suitable polymers can include, for example, polyoxymethylene (POM), polybutylene terephthalate (PBT), polyether block ester, polyether block amide (PEBA), fluorinated ethylene propylene (FEP), polyethylene (PE), polypropylene (PP), polyvinylchloride (PVC), polyurethane, polytetrafluoroethylene (PTFE), polyether-ether ketone (PEEK), polyimide, polyamide, polyphenylene sulfide (PPS), polyphenylene oxide (PPO), polysulfone, nylon, perfluoro(propyl vinyl ether) (PFA), polyether-ester, platinum, polymer/metal composites, etc., or mixtures, blends or combinations thereof, and may also include or be made up of a lubricious polymer having a low coefficient of friction. In some embodiments (not shown), the inner polymer structure 114 includes one or more metals or metal alloys and/or combinations thereof. In a particular embodiment, the inner polymer structure 114 does not include any polymer material and solely comprises a metal and/or metal alloy.

The inner polymer structure 114 can include a single layer of material or it can have two or more layers of the same or different materials. For example, in the embodiment shown in Figure 1B, the inner polymer structure 114 includes a first layer 112 and a second layer 113 surrounding at least a portion of the first layer 112. An inner surface of the first layer 112 defines the shaft lumen 103. The first layer 112 can comprise a lubricious polymer such as HDPE or PTFE, for example, or platinum, PEEK, PE, PP, or a copolymer of tetrafluoroethylene, such as FEP, a copolymer of tetrafluoroethylene with perfluoroethers, such as perfluoroalkoxy alkanes (PFA) (more specifically, perfluoropropyl vinyl ether or perfluoromethyl vinyl ether), or the like. The second layer 113 can be made of any of the materials described above with respect to the inner polymer structure 114 such as, for example, PEBA, PVC, PE, etc. In other embodiments, the inner polymer structure 114 can be formed of a single layer (e.g., only the first layer 112, only the second layer 113, etc.), and in other embodiments the inner polymer structure 114 can include more than two layers (e.g., three layers, four layers, etc.) depending upon the desired characteristics of the catheter 100. In some embodiments the first and second layers 112, 113 have generally the same lengths and are coextensive along the length of the shaft 106, and in other embodiments the first and second layers 112, 113 have different lengths and/or are not coextensive along the shaft 106. For example, in a particular embodiment, the second layer 113 extends along only a portion of the length of the shaft 106 while the first layer 112 extends the entire length (or substantially the entire length) of the shaft 106. In any of the above embodiments, the first layer 112 can have a thickness of about 0.0127 millimeters (about 0.0005 inches) to about 0.127 millimeters (about 0.005 inches), or about 0.0254 millimeters (about 0.001 inches) to about 0.0762 millimeters (about 0.003 inches). Also, in any of the above embodiments, the second layer 113 can have a thickness of about 0.0127 millimeters (about 0.0005 inches) to about 0.127 millimeters (about 0.005 inches), or about 0.0254 millimeters (about 0.001 inches) to about 0.0762 millimeters (about 0.003 inches).

The stiffness of the inner polymer structure 114 can be generally uniform along its length, or the stiffness can vary along its length. The stiffness variation is a function of the size, shape, thickness, and/or materials of the inner polymer structure 114. In embodiments where the stiffness of the inner polymer structure 114 varies along its length, the stiffness can change continuously (e.g., gradually) and/or be stepped from one section to another. In some embodiments, the stiffness of the inner polymer structure 114 decreases in a proximal to distal direction along its length. In other embodiments, the stiffness of the inner polymer structure 114 increases in a proximal to distal direction along it length, and/or increases and decreases in a proximal to distal direction along its length. Additionally, the inner polymer structure 114 can be made of or include a radiopaque material for radiographic visualization. Exemplary radiopaque materials include, for example, gold, platinum, palladium, tantalum, tungsten alloy, polymer materials loaded with radiopaque fillers, and the like. Likewise, in some embodiments, the inner polymer structure 114 is made of or include a material that may aid in MRI imaging, such as, for example, tungsten, Elgiloy, MP35N, nitinol, and others.

In the embodiment shown in Figures 1A-1B, the outer polymer structure 116 directly contacts at least a portion of the inner polymer structure 114 and encases at least a portion of each of the inner braid 160, the outer braid 162, and the coil 170. The outer polymer structure 116 extends distally from the proximal portion 106a of the shaft 106 to a location within the distal portion 106b of the shaft 106 (e.g., the entire length of the shaft 106 or substantially the entire length of the shaft 106). The length of the outer polymer structure 116 can vary depending upon, for example, the length of the shaft 106 and the desired characteristics and functions of the catheter 100. In some embodiments, the outer polymer structure 116 extends substantially the entire length of the shaft 106. In other embodiments, the outer polymer structure 116 extends along only a portion of the length of the shaft 106 and/or has a proximal and/or distal terminus that does not correspond to a proximal terminus and/or distal terminus, respectively, of the shaft 106.

The outer polymer structure 116 (and/or portions thereof) can be made of any suitable polymer (or composites or combinations thereof) and by any suitable process. Suitable polymers can include, for example, polyoxymethylene (POM), polybutylene terephthalate (PBT), polyether block ester, polyether block amide (PEBA), fluorinated ethylene propylene (FEP), polyethylene (PE), polypropylene (PP), polyvinylchloride (PVC), polyurethane, polytetrafluoroethylene (PTFE), polyether-ether ketone (PEEK), polyimide, polyamide, polyphenylene sulfide (PPS), polyphenylene oxide (PPO), polysulfone, nylon, perfluoro(propyl vinyl ether) (PFA), polyether-ester, platinum, polymer/metal composites, etc., or mixtures, blends or combinations thereof. In several embodiments, the outer polymer structure 116 is or at least includes a lubricious polymer. In some embodiments (not shown), the outer polymer structure 116 includes one or more metals or metal alloys (combinations thereof). In a particular embodiment, the outer polymer structure 116 does not include any polymer material and solely comprises a metal and/or metal alloy.

In some embodiments, the stiffness of the outer polymer structure 116 varies along its length. In such embodiments, the stiffness variation may be continuous or stepped by varying the size, shape, thickness, and/or material composition of the outer polymer structure 116. For example, in the embodiment shown in Figures 1A-1B, the outer polymer structure 116 includes four unique portions along its length (labeled proximal to distal as first, second, third and fourth portions 120, 130, 140, and 150, respectively) in which the respective stiffnesses of the portions 120, 130, 140, 150 decrease sequentially in a proximal to distal direction. For example, the first portion 120 has a first stiffness, the second portion 130 has a second stiffness less than the first stiffness, the third portion 140 has a third stiffness less than the second stiffness, and the fourth portion 150 has a fourth stiffness less than the third stiffness. In other embodiments, the stiffness of the outer polymer structure 116 and/or the stiffnesses of the individual portions 120, 130, 140, 150 can increase in a proximal to distal direction (e.g., the second portion 130 can be stiffer than the first portion 120, etc.), increase and decrease in a proximal to distal direction (e.g., the second portion 130 can be stiffer than the first portion 120 but less stiff than the third portion 140, etc.), or be generally uniform in a proximal to distal direction. In other embodiments, the outer polymer structure 116 can have more or fewer portions (e.g., one continuous portion, two portions, three portions, five portions, etc.).

In some embodiments, one or both of the first and second portions 120, 130 can have an individual thickness of about 0.0762 millimeters (about 0.003 inches) to about 0.127 millimeters (about 0.005 inches), and in some embodiments, about 0.1016 millimeters (about 0.004 inches) to about 0.254 millimeters (about 0.010 inches). The fourth portion 150 can have a thickness of about 0.0254 millimeters (about 0.001 inches) to about 0.0762 millimeters (about 0.003 inches). The proximal portion of the tapered portion 192 can have a thickness equivalent to that of the corresponding second portion 130, and the distal portion of the tapered portion 192 can have a thickness generally equivalent to that of the corresponding fourth portion 150. Thus, the third portion 140 can have a proximal thickness between about 0.0762 millimeters (about 0.003 inches) to about 0.127 millimeters (about 0.005 inches), or in some embodiments about 0.1016 millimeters (about 0.004 inches) to about 0.254 millimeters (about 0.010 inches), and a distal portion have a thickness of about 0.0254 millimeters (about 0.001 inches) to about 0.0762 millimeters (about 0.003 inches).

The portions 120, 130, 140, 150, either individually or any combination thereof, can be made of the same or different materials, have the same or different size, have the same or different thickness, and/or have the same or different cross-sectional shape. In some embodiments, the outer polymer structure 116 can include two or more layers (e.g., an inner layer surrounding an outer layer, etc.), and each layer can have the same or different material compositions, thicknesses, and/or stiffnesses. Additionally, the portions 120, 130, 140, 150, either individually or any combination thereof, can have a uniform or varying stiffness along its respective length. In other words, the portions 120, 130, 140, 150, either individually or any combination thereof, can have a uniform or varying size, shape, thickness, and/or material composition along its respective length. For example, in the embodiment shown in Figure 1B, each of the portions 120, 130, 140, 150 has a constant material composition and cross-sectional shape along its respective length. Each of the first, second, and fourth portions 120, 130, 150 also has a generally constant thickness along its respective length; accordingly, each of the first, second, and fourth portions 120, 130, 150 has a generally constant stiffness along its respective length. The third portion 140, however, includes the tapered portion 192 (Figure 1A) and thus varies in thickness (and stiffness) along its length. In other embodiments, the third portion 140 does not coincide with the tapered portion 192 and/or the tapered portion 192 spans more than one of the portions 120, 130, 140, 150.

It will be appreciated that while the inner polymer structure 114 and the outer polymer structure 116 are described herein as separate components with respect to the illustrated embodiments, the inner and outer polymer structures 114, 116 can be provided as a single layer or structure. For example, the inner polymer structure 114 and outer polymer structure 116 may be provided separately, but attached or combined together to physically form a single layer (e.g., a single homogeneous material).

Referring still to the embodiment shown in Figure 1B, the inner braid 160 is on and around the inner polymer structure 114, and the outer polymer structure 116 is on and around the inner braid 160. In some embodiments, the inner braid 160 directly contacts at least a portion of both the inner polymer structure 114 and the outer polymer structure 116. In other embodiments, the outer polymer structure 116 is between at least a portion of the inner polymer structure 114 and at least a portion of the inner braid 160. In the embodiment shown in Figures 1A-1B, the inner braid 160 extends distally from the proximal portion 106a of the shaft 106 to a distal terminus 160b aligned with or just proximal of the distal terminus of the shaft 106. In other embodiments, the inner braid 160 extends the entire length of the shaft 106. The length of the inner braid 160 can vary depending upon, for example, the length of the shaft 106 and the desired characteristics and functions of the catheter 100.

In some embodiments, at least a portion of the inner braid 160 is coextensive with at least a portion of the outer braid 162. For example, in the embodiment shown in Figure 1B, the inner braid 160 has a distal terminus 160b located at a position along the shaft 106 distal of a proximal terminus (not shown) of the outer braid 162 and proximal of a distal terminus 162b of the outer braid 162. In other embodiments (not shown), no portion of the inner braid 160 is coextensive with a portion of the outer braid 162. Additionally, in some embodiments at least a portion of the inner braid 160 is coextensive with at least a portion of the coil 170, and in other embodiments the inner braid 160 is adjacent to and/or spaced apart from the coil 170 along the length of the shaft 106. For example, in the embodiment shown in Figure 1B, the distal terminus 160b of the inner braid 160 is located at a position along the shaft 106 proximal of a proximal terminus 170a of the coil 170 such that no portion of the inner braid 160 is coextensive with any portion of the coil 170. Alternatively, in some embodiments (not shown) the distal terminus 160b of the inner braid 160 is located at a position along the shaft 106 distal of a proximal terminus 170a of the coil 170 such that at least a portion of the inner braid 160 is coextensive with at least a portion of the coil 170.

In the embodiment shown in Figures 1A-1B, the outer braid 162 is around the inner braid 160, and the outer polymer structure 116 contacts the outer braid 162. In some embodiments the outer braid 162 directly contacts the inner braid 160. In other embodiments, the outer polymer structure 116 is between at least a portion of the inner braid 160 and at least a portion of the outer braid 162. In the embodiment shown in Figure 1B, a distal portion of the outer braid 162 is around a proximal portion of the coil 170. In some embodiments the outer braid 162 directly contacts the coil 170. In other embodiments, the outer polymer structure 116 is between at least a portion of the outer braid 162 and at least a portion of the coil 170.

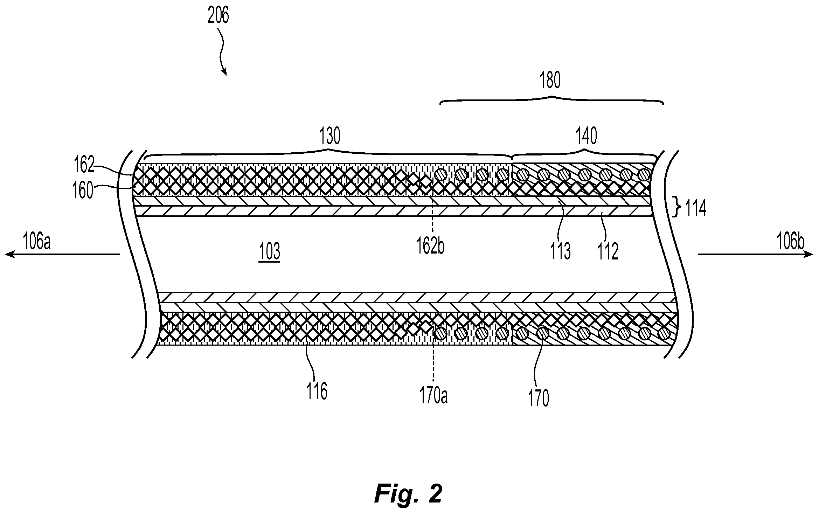

The outer braid 162 extends distally from the proximal portion 106a of the shaft 106 to a distal terminus 162b proximal to the distal terminus of the shaft 106. In other embodiments, the outer braid 162 extends the entire length of the shaft 106. The length of the outer braid 162 can vary depending upon, for example, the length of the shaft 106 and the desired characteristics and functions of the catheter 100. In some embodiments, at least a portion of the outer braid 162 is coextensive with at least a portion of the coil 170. For example, in the embodiment shown in Figure 1B, the distal terminus 162b of the outer braid 162 is located at a position along the shaft 106 that is distal of the proximal terminus 170a of the coil 170. In those embodiments where at least a portion of the outer braid 162 is coextensive with at least a portion of the coil 170, the coextensive portions of the outer braid 162 and the coil 170 form an overlapping region 180. As shown in Figure 1B, in some embodiments the outer braid 162 surrounds the coil 170 within the overlapping region 180. In other embodiments, the coil 170 surrounds the outer braid 162 within the overlapping region 180 (Figure 2, described in greater detail below). In yet other embodiments, the outer braid 162 is spaced apart from and/or adjacent to the coil 170 such that no portion of the outer braid 162 is coextensive with any portion of the coil 170.

The inner braid 160 and/or the outer braid 162 can individually have a generally uniform pitch along its respective length or may have a varying pitch along its respective length. The flexibility of the individual inner braid 160 and/or the outer braid 162 may vary continuously along its respective length by continuously varying the pitch or may vary along its respective length in a stepwise fashion by stepwise varying the pitch. Moreover, the inner braid 160 and/or the outer braid 162 can individually have a generally constant braid angle along its respective length or have a varying braid angle along its respective length to provide different zones of stiffness and/or flexibility. The inner braid 160 and/or the outer braid 162 can be formed of braided filaments having the same or varying diameters (individually and/or relative to the other braid). In some embodiments, the inner braid 160 and/or the outer braid 162 are further shaped using a heat setting process. Additionally, the inner braid 160 and the outer braid 162 can have the same or different pitch, stiffness, braid angle, filament diameters, and filament count. In some embodiments, the inner and/or outer braids 160, 162 individually have a pitch of 17.71654 picks per centimeter (45 picks per inch (PPI) to 31.49606 picks per centimeter (80 PPI). In a particular embodiment, the shaft 106 includes a single braid. Additionally, in some embodiments, the inner braid 160 and/or the outer braid 162 can be made of or include a radiopaque or imaging material.

The inner 160 and/or outer braids 162 are formed of a plurality of interwoven wires. The wires can have a circular or rectangular cross-sectional shape. The wires can be made of one or more metals, such as stainless steel, platinum, silver, tantalum, and the like. In some embodiments, the wires can include or be made of non-metallic materials. In some embodiments, the wires are made of a superelastic or shape-memory material, such as nitinol. For those embodiments utilizing wires having a rectangular shape, the wires can have a cross-sectional area of about 0.0127 millimeters (about 0.0005 inches) by 0.0635 millimeters (0.0025 inches) to about 0.0254 millimeters (about 0.001 inches) by 0.127 millimeters (0.005 inches).

The coil 170 can be one or more round wires or flat ribbons helically wound around the inner polymer structure 114. In the embodiment shown in Figures 1A-1B, the outer polymer structure 116 encases the coil 170. The proximal terminus 170a of the coil 170 is positioned along the distal portion 106b of the shaft 106, and the distal terminus 170b of the coil 170 is positioned generally in alignment with or just proximal to the distal terminus of the shaft 106. Accordingly, the coil 170 is completely disposed within the distal portion of the shaft. In other embodiments, at least a portion of the coil 170 is outside of the distal portion 106b of the shaft 106. The pitch of adjacent turns of the coil 170 may be tightly wound so that each turn touches the succeeding turn or the pitch may be set such that the coil 170 is wound in an open fashion. The pitch of the coil 170 can be the same or may vary along the length of the coil 170. The coil 170 can have a pitch of about 0.1016 millimeters (about 0.004 inches) to about 0.3556 millimeters (about 0.014 inches). In some embodiments, the pitch of the coil 170 depends on the inner diameter of the shaft 106. For example, for a shaft inner diameter of about 0.4318 millimeters (about 0.017 inches), the coil 170 can have a pitch of about 0.1016 millimeters (about 0.004 inches) to about 0.2286 millimeters (about 0.009 inches). For a shaft inner diameter of about 0.5334 millimeters (about 0.021 inches), the coil 170 can have a pitch of about 0.1524 millimeters (about 0.006 inches) to about 0.2794 millimeters (about 0.011 inches). For a shaft inner diameter of about 0.6858 millimeters (about 0.027 inches), the coil 170 can have a pitch of about 0.1778 millimeters (about 0.007 inches) to about 0.3048 millimeters (about 0.012 inches). For a shaft inner diameter of about 0.1143 millimeters (about 0.0045 inches), the coil 170 can have a pitch of about 0.254 millimeters (about 0.010 inches) to about 0.3556 millimeters (about 0.014 inches). Additionally, in some embodiments, the coil 170 or portions thereof can be made of or include a radiopaque or imaging material.

The wire of the coil 170 can be made of one or more metals, such as stainless steel, platinum, silver, tantalum, and the like. In other embodiments, the wire of the coil 170 can include or be made of non-metallic materials. In a particular embodiment, the wires are made of a superelastic or shape-memory material, such as nitinol The wire can have an outer diameter of about 0.0254 millimeters (about 0.001 inches) to about 0.127 millimeters (about 0.005 inches), or in some embodiments about 0.0254 millimeters (about 0.001 inches) to about 0.0762 millimeters (about 0.003 inches).

It will be appreciated that the inner braid 160, outer braid 162, and coil 170 can have other suitable configurations and/or relative positions along the length of the shaft 106. For example, in some embodiments the inner braid 160 can be coextensive with at least a portion of the coil 170, and in some embodiments the inner braid 160 can be generally coextensive with the outer braid 162. In a particular embodiment, at least a portion of the outer braid 162 is not coextensive with a portion of the coil 170.

Figure 2 is a cross-sectional side view of a portion of a catheter shaft 206 configured in accordance with another embodiment of the present technology. The shaft 206 can be generally similar to the shaft 106 shown in Figures 1A-1B, except the coil 170 in the shaft 206 of Figure 2 surrounds the outer braid 162 within the overlapping region 180.

### II. Selected Embodiments of Distal Portions of Catheter Shafts of the Present Technology

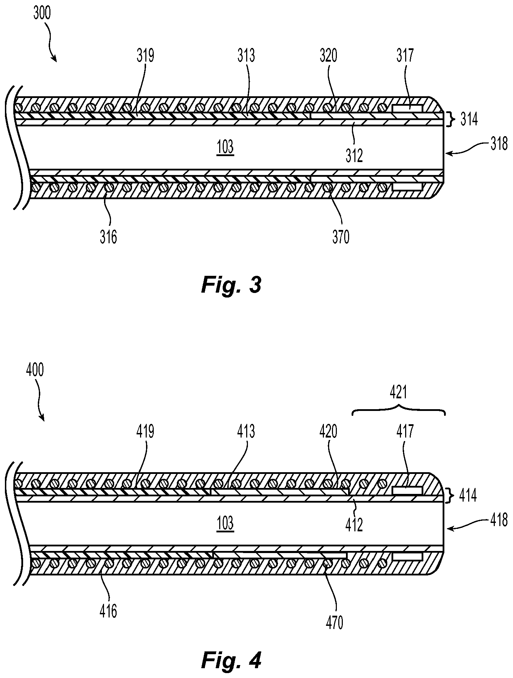

Figures 3-8 are cross-sectional side views of distal portions of catheter shafts configured in accordance with the present technology. Any of the distal portions (or aspects thereof) described below can be combined with any of the catheter shafts described above with reference to Figures 1A-2. As described in greater detail below, the distal portion embodiments of the present technology include regions of varying stiffness and/or preferential bending that provide improved bending/buckling at the distal portion when contacting the wall of tortuous vessels, thereby improving ease of navigation of the corresponding shaft and/or distal portion.

Figure 3 is a cross-sectional side view of a distal portion 300 of a catheter shaft configured in accordance with the present technology. The distal portion 300 can include a radiopaque marker 317, an inner polymer structure 314, an outer polymer structure 316 surrounding at least a portion of the inner polymer structure 314, and a coil 370 wrapped around at least a portion of the inner polymer structure 314. As shown in Figure 3, the inner polymer structure 314 extends the length of the distal portion 300 such that the inner polymer structure 314 terminates distally at an opening 318 at the distal terminus of the distal portion 300. The inner polymer structure 314 defines a lumen that can be generally continuous with the lumen 103 of any of the shaft embodiments described above with reference to Figures 1A-2.

The inner polymer structure 314 can include a single layer of material or it can have two or more layers of the same or different materials. For example, in the embodiment shown in Figure 3, the inner polymer structure 314 includes a first layer 312 and a second layer 313 surrounding the first layer 312. Accordingly, an inner surface of the first layer 312 defines the shaft lumen 103 at the distal portion 300. The first layer 312 can comprise a lubricious polymer such as HDPE or PTFE, for example, or platinum, PEEK, PE, PP, or a copolymer of tetrafluoroethylene, such as FEP, a copolymer of tetrafluoroethylene with perfluoroethers, such as PFA (more specifically, perfluoropropyl vinyl ether or perfluoromethyl vinyl ether), or the like. The second layer 313 can be made of any of the materials described above with respect to the inner polymer structure 114. Moreover, in some embodiments the inner polymer structure 314 can be formed of a single layer (e.g., only the first layer 312, only the second layer 313, etc.), and in other embodiments the inner polymer structure 314 can include more than two layers (e.g., three layers, four layers) depending on the desired characteristics of the distal portion 300 of the catheter.

The stiffness of the inner polymer structure 314 can be generally uniform along its length, or the stiffness can vary along its length. In the embodiment shown in Figure 3, the second layer 313 of the inner polymer structure 314 includes two unique portions along its length (labeled proximal to distal as first portion 319 and second portion 320). The first and second portions 319, 320 can have at least one of a different size, shape, thickness, and material composition such that the first portion 319 has a different stiffness than the second portion 320 (or in other words, the second portion 320 is softer than the first portion 319). For example, the first portion 319 can be a first material and the second portion 320 can be a second material different than the first material such that a stiffness of the first portion 319 is greater than a stiffness of the second portion 320. In other embodiments, a stiffness of the inner polymer structure 314 can increase in a proximal to distal direction along its length, or increase and decrease in a proximal to distal direction along its length. For example, in a particular embodiment, the second portion 320 can have a stiffness that is greater than or equal to the stiffness of the first portion 319. In other embodiments, the inner polymer structure 314 can have more or fewer portions (e.g., one continuous portion, three portions, four portions, etc.).

In the embodiment shown in Figure 3, both the first and second layers 312, 313 of the inner polymer structure 314 extend along the entire length of the distal portion 300 such that the distal termini of both the first and second layers 312, 313 are at the distal terminus of the distal portion 300. Additionally, the second portion 319 of the second layer 313 defines a portion of the distal terminus of the distal portion 300 of the shaft. As such, the distal-most surfaces of both the inner and the outer polymer structures 314, 316 define the distal terminus of the distal portion 300 of the shaft. In other embodiments, the first layer 312 terminates proximal to the distal terminus of the distal portion 300.

Although the inner polymer structure 314 is shown having two portions 319, 320 in Figure 3, in other embodiments the inner polymer structure 314 can have a single continuous portion or more than two portions (e.g., three portions, four portions, etc.). Moreover, although the second layer 313 is shown having multiple portions, in other embodiments the first layer 312 can additionally or alternatively include multiple portions.

Referring still to the embodiment shown in Figure 3, the outer polymer structure 316 directly contacts at least a portion of the inner polymer structure 314 and encases at least a portion of the coil 370. For example, in the embodiment shown in Figure 3, at least a portion of the surface of the coil 370 directly contacts the first and second portions 319, 320 of the second layer 313 of the inner polymer structure 314, while a remaining portion of the coil's surface directly contacts the outer polymer structure 316. Additionally, the outer polymer structure 316 extends along the length of the distal portion 300 such that a distal terminus of the outer polymer structure 316 corresponds to the distal terminus of the distal portion 300. In other embodiments, the outer polymer structure 316 extends along only a portion of the length of the distal portion 300 and/or has a proximal and/or distal terminus that does not correspond to a proximal terminus and/or distal terminus, respectively, of the distal portion 300. Moreover, the outer polymer structure 316 (and/or portions thereof) can be made of any of the materials described above with respect to the outer polymer structure 116.

The coil 370 can be one or more round wires or flat ribbons helically wound around the inner polymer structure 314, and the outer polymer structure 316 can encase at least a portion of the coil 370. The coil 370 can extend all or a portion of the length of the distal portion 300. For example, in the embodiment shown in Figure 3, the coil 370 has a distal terminus that is aligned with or just proximal of the radiopaque marker 317, and the radiopaque marker 317 is proximal of the distal terminus of the distal portion 300. As such, a distal terminus of the coil 370 is spaced apart from a distal terminus of the shaft. The pitch of adjacent turns of the coil 370 may be tightly wound so that each turn touches the succeeding turn or the pitch may be set such that the coil 370 is wound in an open fashion. The pitch of the coil 370 can be the same or vary along the length of the coil 370. Additionally, in some embodiments, the coil 370 or portions thereof can be made of or include a radiopaque or imaging material.

Figure 4 is a cross-sectional side view of a distal portion 400 of a catheter shaft configured in accordance with another embodiment of the present technology. The distal portion 400 can include a radiopaque marker 417, an inner polymer structure 414, an outer polymer structure 416 surrounding at least a portion of the inner polymer structure 414, and a coil 470 wound around at least a portion of the inner polymer structure 414. The inner polymer structure 414 defines a lumen that can be generally continuous with the lumen 103 of any of the shaft embodiments described above with reference to Figures 1A-2.

The inner polymer structure 414 can include a single layer of material or it can have two or more layers of the same or different materials. For example, in the embodiment in Figure 4, the inner polymer structure 414 includes a first layer 412 and a second layer 413 surrounding the first layer 412. Accordingly, an inner surface of the first layer 412 defines the shaft lumen 103 at the distal portion 400. The first layer 412 can comprise a lubricious polymer such as HDPE or PTFE, for example, or platinum, PEEK, PE, PP, or a copolymer of tetrafluoroethylene, such as FEP, a copolymer of tetrafluoroethylene with perfluoroethers, such as PFA (more specifically, perfluoropropyl vinyl ether or perfluoromethyl vinyl ether), or the like. The second layer 413 can be made of any of the materials described above with respect to the inner polymer structure 414. Moreover, in some embodiments the inner polymer structure 414 can be formed of a single layer (e.g., only the first layer 412, only the second layer 413, etc.), and in other embodiments the inner polymer structure 414 can include more than two layers (e.g., three layers, four layers) depending upon the desired characteristics of the catheter.

The stiffness of the inner polymer structure 414 can be generally uniform along its length, or the stiffness can vary along its length. In the embodiment shown in Figure 4, the second layer 413 of the inner polymer structure 414 includes two unique portions (labeled proximal to distal as first portion 419 and second portion 420) adjacent one another along its length having different stiffnesses. The first and second portions 419, 420 can have at least one of a different size, shape, thickness, and material composition such that the first portion 419 has a different stiffness than the second portion 420. For example, the first portion 419 can be a first material and the second portion 420 can be a second material different than the first material such that a stiffness of the first portion 419 is greater than a stiffness of the second portion 420. In other embodiments, a stiffness of the inner polymer structure 414 can increase in a proximal to distal direction along its length, or increase and decrease in a proximal to distal direction along its length. For example, in a particular embodiment, the second portion 420 has a stiffness that is greater than or equal to the stiffness of the first portion 419.

In the embodiment shown in Figure 4, the second layer 413 of the inner polymer structure 414 extends along only a portion of the length of the distal portion 400 such that a distal terminus of the second layer 413 is proximal of the distal terminus of the outer polymer structure 416 and the distal terminus of the distal portion 400. Accordingly, in contrast to the embodiment shown in Figure 3, only the distal-most portions of the outer polymer structure 416 and the first layer 312 define the distal terminus of the distal portion 400 of the shaft (and not the second layer 313). Likewise, a distal region 421 of the distal portion 400 does not include the second layer 413 and comprises only the first layer 412, the outer polymer structure 416, the radiopaque marker 417, and a portion of the coil 470. Accordingly, the distal region 421 is more flexible than the remainder of the distal portion 400. In some embodiments, the first layer 412 and/or the coil 470 terminates proximal of the distal region 421 such that the distal region 412 comprises the first layer 412 and the outer polymer structure 416. The length of the distal region 421 can be between about 0.5 mm and about 5 cm.

Although the inner polymer structure 414 is shown having two portions 419, 420 in Figure 4, in other embodiments the inner polymer structure 414 can have a single continuous portion or more than two portions (e.g., three portions, four portions, etc.). Moreover, although the second layer 413 is shown having multiple portions, in other embodiments the first layer 412 can additionally or alternatively include multiple portions.

Referring still to the embodiment shown in Figure 4, the outer polymer structure 416 directly contacts at least a portion of the inner polymer structure 414 and encases at least a portion of the coil 470. For example, in the embodiment shown in Figure 4, at least a portion of the surface of the coil 470 directly contacts the inner polymer structure 414, while a remaining portion of the coil's surface directly contacts the outer polymer structure 416. As shown in Figure 4, in some embodiments the outer polymer structure 416 extends along the length of the distal portion 400 such that a distal terminus of the outer polymer structure 416 corresponds to the distal terminus of the distal portion 400. The outer polymer structure 416 (and/or portions thereof) can be made of any of the materials described above with respect to the outer polymer structure 116.

The coil 470 can be one or more round wires or flat ribbons helically wound around the inner polymer structure 414. The coil 470 can extend all or a portion of the length of the distal portion 400. For example, in the embodiment shown in Figure 4, the coil 470 has a distal terminus that is aligned with or just proximal of the radiopaque marker 417, and the radiopaque marker 417 is proximal of the distal terminus of the distal portion 400. The pitch of adjacent turns of the coil 470 may be tightly wound so that each turn touches the succeeding turn or the pitch may be set such that the coil 470 is wound in an open fashion. The pitch of the coil 470 can be the same or vary along the length of the coil 470. Additionally, in some embodiments, the coil 470 or portions thereof can be made of or include a radiopaque or imaging material.

The distal portions 300/400 provide several advantages over distal portions of conventional catheters, especially microcatheters for delivering occlusive devices (such as coils) to cerebral aneurysms. For example, the distal portions 300 and 400 have a (relatively) softer distal tip and a (relatively) stiffer region immediately adjacent and proximal to the softer distal tip. Such a construction allows for improved bending and trackability at the distal tip bend (for positioning at the aneurysm neck) while the proximal stiffer region of the distal portion 300/400 provides additional support and stability to the distal portion 300/400, thereby lessening or preventing kickback of the shaft during deployment of an occlusive device (such as a coil) in an aneurysm.

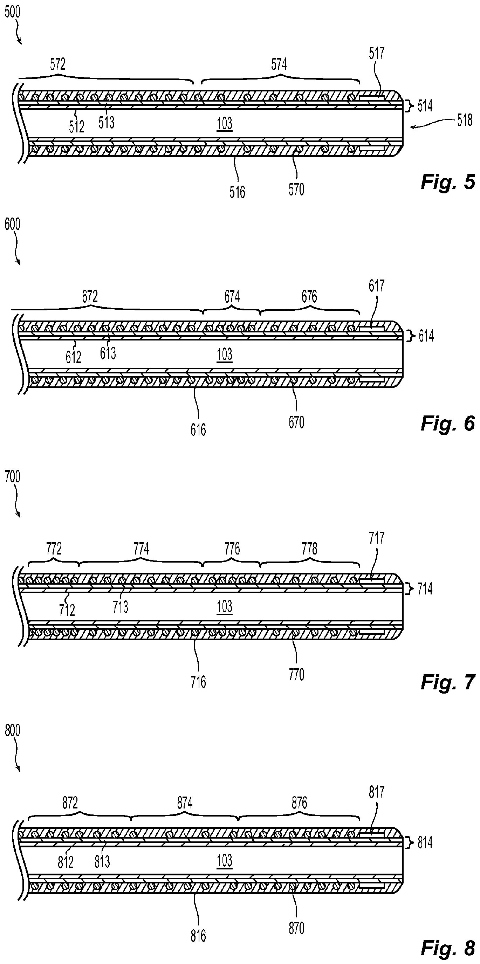

Figure 5 is a cross-sectional side view of a distal portion 500 of a catheter shaft configured in accordance with another embodiments of the present technology. The distal portion 500 can include a radiopaque marker 517, an inner polymer structure 514, an outer polymer structure 516 surrounding at least a portion of the inner polymer structure 514, and a coil 570 wound around at least a portion of the inner polymer structure 514. In the embodiment shown in Figure 5, the inner polymer structure 514 extends the length of the distal portion 500 such that the inner polymer structure 514 terminates distally at an opening 518 at the distal terminus of the distal portion 500. The inner polymer structure 514 defines a lumen that can be generally continuous with the lumen 103 of any of the shaft embodiments described above with reference to Figures 1A-2.

The inner polymer structure 514 can include a single layer of material or it can have two or more layers of the same or different materials. For example, in the embodiment shown in Figure 5, the inner polymer structure 514 includes a first layer 512 and a second layer 513 surrounding the first layer 512. As such, an inner surface of the first layer 512 defines the shaft lumen 103. The second layer 513 can be made of any of the materials described above with respect to the inner polymer structure 514. The first layer 512 can comprise a lubricious polymer such as HDPE or PTFE, for example, or platinum, PEEK, PE, PP, or a copolymer of tetrafluoroethylene, such as FEP, a copolymer of tetrafluoroethylene with perfluoroethers, such as PFA (more specifically, perfluoropropyl vinyl ether or perfluoromethyl vinyl ether), or the like. Moreover, in some embodiments the inner polymer structure 514 can be formed of a single layer (e.g., only the first layer 512, only the second layer 513, etc.), and in other embodiments the inner polymer structure 514 can include more than two layers (e.g., three layers, four layers) depending upon the desired characteristics of the device.

The outer polymer structure 516 directly contacts at least a portion of the inner polymer structure 514 and encases at least a portion of the coil 570. For example, in the embodiment shown in Figure 5, at least a portion of the surface of the coil 570 directly contacts the second layer 513 of the inner polymer structure 514, while a remaining portion of the coil's surface directly contacts the outer polymer structure 516. As shown in Figure 5, in some embodiments the outer polymer structure 516 extends along the length of the distal portion 500 such that a distal terminus of the outer polymer structure 516 corresponds to the distal terminus of the distal portion 500. The outer polymer structure 516 (and/or portions thereof) can be made of any of the materials described above with respect to the outer polymer structure 116.

The coil 570 can be one or more round wires or flat ribbons helically wound around the inner polymer structure 514. The coil 570 can extend all or a portion of the length of the distal portion 500. For example, in the embodiment shown in Figure 5, the coil 570 has a distal terminus that is aligned with or just proximal of the radiopaque marker 517, and the radiopaque marker 517 is proximal of the distal terminus of the distal portion 500. The pitch of adjacent turns of the coil 570 may be tightly wound so that each turn touches the succeeding turn or the pitch may be set such that the coil 570 is wound in an open fashion. The pitch of the coil 570 can be the same or vary along the length of the coil 570. For example, in the embodiment shown in Figure 5, the coil 570 has a first portion 572 and a second portion 574 distal of the first portion 572. The first portion 572 has a first pitch and the second portion 574 has a second pitch that is greater than the first pitch. Accordingly, a length of the distal portion 500 corresponding to the first portion 572 of the coil 570 is less flexible than a length of the distal portion 500 corresponding to the second portion 574 of the coil 570. Additionally, in some embodiments, the coil 570 or portions thereof can be made of or include a radiopaque or imaging material.

Figure 6 is a cross-sectional side view of a distal portion 600 of a catheter shaft configured in accordance with the present technology. The distal portion 600 can include a radiopaque marker 617, an inner polymer structure 614, an outer polymer structure 616 surrounding at least a portion of the inner polymer structure 614, and a coil 670 wound around at least a portion of the inner polymer structure 614. In the embodiment shown in Figure 6, the inner polymer structure 614 extends the length of the distal portion 600 such that the inner polymer structure 614 terminates distally at an opening 618 at the distal terminus of the distal portion 600. The inner polymer structure 614 defines a lumen that can be generally continuous with the lumen 103 of any of the shaft embodiments described above with reference to Figures 1A-2.

The inner polymer structure 614 can include a single layer of material or it can have two or more layers of the same or different materials. For example, as shown in Figure 6, the inner polymer structure 614 can include a first layer 612 and a second layer 613 surrounding the first layer 612. As such, an inner surface of the first layer 612 defines the shaft lumen 103. The second layer 613 can be made of any of the materials described above with respect to the inner polymer structure 614. The first layer 612 can include a lubricious polymer such as HDPE or PTFE, for example, or a copolymer of tetrafluoroethylene with perfluoroalkyl vinyl ether (PFA) (more specifically, perfluoropropyl vinyl ether or perfluoromethyl vinyl ether), or the like. Moreover, in some embodiments the inner polymer structure 614 can be formed of a single layer (e.g., only the first layer 612, only the second layer 613, etc.), and in other embodiments the inner polymer structure 614 can include more than two layers (e.g., three layers, four layers) depending upon the desired characteristics of the device.

The outer polymer structure 616 directly contacts at least a portion of the inner polymer structure 614 and encases at least a portion of the coil 670. For example, in the embodiment shown in Figure 6, at least a portion of the surface of the coil 670 directly contacts the second layer 613 of the inner polymer structure 614, while a remaining portion of the coil's surface directly contacts the outer polymer structure 616. In some embodiments the outer polymer structure 616 extends along the length of the distal portion 600 such that a distal terminus of the outer polymer structure 616 corresponds to the distal terminus of the distal portion 600. The outer polymer structure 616 (and/or portions thereof) can be made of any of the materials described above with respect to the outer polymer structure 116.

The coil 670 can be one or more round wires or flat ribbons helically wound around the inner polymer structure 614. The coil 670 can extend all or a portion of the length of the distal portion 600. For example, in the embodiment shown in Figure 6, the coil 670 has a distal terminus that is aligned with or just proximal of the radiopaque marker 617, and the radiopaque marker 617 is proximal of the distal terminus of the distal portion 600. The pitch of adjacent turns of the coil 670 may be tightly wound so that each turn touches the succeeding turn or the pitch may be set such that the coil 670 is wound in an open fashion. The pitch of the coil 670 can be the same or vary along the length of the coil 670. For example, in the embodiment shown in Figure 6, the coil 670 has a first portion 672, a second portion 674 distal of the first portion 672, and a third portion 676 distal of the second portion 674. The first portion 672 has a first pitch, the second portion 674 has a second pitch less than the first pitch, and the third portion 676 has a third pitch greater than the second pitch. Accordingly, regions of the distal portion 600 corresponding to the first and third portions 672, 676 of the coil 670 are more flexible than a region of the distal portion 600 corresponding to the second portion 674 of the coil 670. In some embodiments, the first and third pitches can be the same or different so long as the average pitch of the first and third portions 672, 676 is less than the average pitch of the second portion 674. Additionally, in some embodiments, the coil 670 or portions thereof can be made of or include a radiopaque or imaging material.

Figure 7 is a cross-sectional side view of a distal portion 700 of a catheter shaft configured in accordance with the present technology. The distal portion 700 can include a radiopaque marker 717, an inner polymer structure 714, an outer polymer structure 716 surrounding at least a portion of the inner polymer structure 714, and a coil 770 wound around at least a portion of the inner polymer structure 714. In the embodiment shown in Figure 7, the inner polymer structure 714 extends the length of the distal portion 700 such that the inner polymer structure 714 terminates distally at an opening 718 at the distal terminus of the distal portion 700. The inner polymer structure 714 defines a lumen that can be generally continuous with the lumen 103 of any of the shaft embodiments described above with reference to Figures 1A-2.

The inner polymer structure 714 can include a single layer of material or it can have two or more layers of the same or different materials. For example, as shown in Figure 7, the inner polymer structure 714 can include a first layer 712 and a second layer 713 surrounding the first layer 712. As such, an inner surface of the first layer 712 defines the shaft lumen 103. The second layer 713 can be made of any of the materials described above with respect to the inner polymer structure 714. The first layer 712 can comprise a lubricious polymer such as HDPE or PTFE, for example, or platinum, PEEK, PE, PP, or a copolymer of tetrafluoroethylene, such as FEP, a copolymer of tetrafluoroethylene with perfluoroethers, such as PFA (more specifically, perfluoropropyl vinyl ether or perfluoromethyl vinyl ether), or the like. Moreover, in some embodiments the inner polymer structure 714 can be formed of a single layer (e.g., only the first layer 712, only the second layer 713, etc.), and in other embodiments the inner polymer structure 714 can include more than two layers (e.g., three layers, four layers) depending upon the desired characteristics of the device.

The outer polymer structure 716 directly contacts at least a portion of the inner polymer structure 714 and encases at least a portion of the coil 770. For example, in the embodiment shown in Figure 7, at least a portion of the surface of the coil 770 directly contacts the second layer 713 of the inner polymer structure 714, while a remaining portion of the coil's surface directly contacts the outer polymer structure 716. In some embodiments the outer polymer structure 716 extends along the length of the distal portion 700 such that a distal terminus of the outer polymer structure 716 corresponds to the distal terminus of the distal portion 700. The outer polymer structure 716 (and/or portions thereof) can be made of any of the materials described above with respect to the outer polymer structure 116.

The coil 770 can be one or more round wires or flat ribbons helically wound around the inner polymer structure 714. The coil 770 can extend all or a portion of the length of the distal portion 700. For example, in the embodiment shown in Figure 7, the coil 770 has a distal terminus that is aligned with or just proximal of the radiopaque marker 717, and the radiopaque marker 717 is proximal of the distal terminus of the distal portion 700. The pitch of adjacent turns of the coil 770 may be tightly wound so that each turn touches the succeeding turn or the pitch may be set such that the coil 770 is wound in an open fashion. The pitch of the coil 770 can be the same or vary along the length of the coil 770. For example, in the embodiment shown in Figure 7, the coil 770 has a first portion 772, a second portion 774 distal of the first portion 772, a third portion 776 distal of the second portion 774, and a fourth portion 778 distal of the third portion 776. The first portion 772 has a first pitch, the second portion 774 has a second pitch greater than the first pitch, the third portion 776 has a third pitch less than the second pitch, and the fourth portion 778 has a fourth pitch greater than each of the first and third pitches. Accordingly, regions of the distal portion 700 corresponding to the first and third portions 772, 776 of the coil 770 are less flexible than regions of the distal portion 700 corresponding to the second and fourth portions 774, 778 of the coil 770.

In some embodiments, the first and third pitches can be generally the same, and the second and fourth pitches can be generally the same and greater than the first and third pitches. In other embodiments, the first and third portions 772, 776 can have the same and/or different pitches and/or the second and fourth portions 774, 778 can have the same and/or different pitches, so long as the average pitch of the first and third portions 772, 776 is less than the average pitch of the second and fourth portions 774, 778. Additionally, in some embodiments, the coil 770 or portions thereof can be made of or include a radiopaque or imaging material.

Figure 8 is a cross-sectional side view of a distal portion 800 of a catheter shaft configured in accordance with the present technology. The distal portion 800 can include a radiopaque marker 817, an inner polymer structure 814, an outer polymer structure 816 surrounding at least a portion of the inner polymer structure 814, and a coil 870 wound around at least a portion of the inner polymer structure 814. In the embodiment shown in Figure 8, the inner polymer structure 814 extends the length of the distal portion 800 such that the inner polymer structure 814 terminates distally at an opening 818 at the distal terminus of the distal portion 800. The inner polymer structure 814 defines a lumen that can be generally continuous with the lumen 103 of any of the shaft embodiments described above with reference to Figures 1A-2.

The inner polymer structure 814 can include two or more layers. For example, as shown in Figure 8, the inner polymer structure 814 can include a first layer 812 and a second layer 813 surrounding the first layer 812. As such, an inner surface of the first layer 812 defines the shaft lumen 103. The second layer 813 can be made of any of the materials described above with respect to the inner polymer structure 814. The first layer 812 can comprise a lubricious polymer such as HDPE or PTFE, for example, or platinum, PEEK, PE, PP, or a copolymer of tetrafluoroethylene, such as FEP, a copolymer of tetrafluoroethylene with perfluoroethers, such as PFA (more specifically, perfluoropropyl vinyl ether or perfluoromethyl vinyl ether), or the like. Moreover, in some embodiments the inner polymer structure 814 can be formed of a single layer (e.g., only the first layer 812, only the second layer 813, etc.), and in other embodiments the inner polymer structure 814 can include more than two layers (e.g., three layers, four layers) depending upon the desired characteristics of the device.

The outer polymer structure 816 directly contacts at least a portion of the inner polymer structure 814 and encases at least a portion of the coil 870. For example, in the embodiment shown in Figure 8, at least a portion of the surface of the coil 870 directly contacts the second layer 813 of the inner polymer structure 814, while a remaining portion of the coil's surface directly contacts the outer polymer structure 816. In some embodiments the outer polymer structure 816 extends along the length of the distal portion 800 such that a distal terminus of the outer polymer structure 816 corresponds to the distal terminus of the distal portion 800. The outer polymer structure 816 (and/or portions thereof) can be made of any of the materials described above with respect to the outer polymer structure 116.

The coil 870 can be one or more round wires or flat ribbons helically wound around the inner polymer structure 814.The coil 870 can extend all or a portion of the length of the distal portion 800. For example, in the embodiment shown in Figure 8, the coil 870 has a distal terminus that is aligned with or just proximal of the radiopaque marker 817, and the radiopaque marker 817 is proximal of the distal terminus of the distal portion 800. The pitch of adjacent turns of the coil 870 may be tightly wound so that each turn touches the succeeding turn or the pitch may be set such that the coil 870 is wound in an open fashion. The pitch of the coil 870 can be the same or vary along the length of the coil 870. For example, in the embodiment shown in Figure 8, the coil 870 has a first portion 872, a second portion 874 distal of the first portion 872, and a third portion 876 distal of the second portion 874. The first portion 872 has a first pitch, the second portion 874 has a second pitch greater than the first pitch, and the third portion 876 has a third pitch less than the second pitch. Accordingly, regions of the distal portion 800 corresponding to the first and third portions 872, 876 of the coil 870 are less flexible than a region of the distal portion 800 corresponding to the second portion 874 of the coil 870. In some embodiments, the first and third pitches can be the same or different so long as the average pitch of the first and third portions 872, 876 is less than the average pitch of the second portion 874. Additionally, in some embodiments, the coil 870 or portions thereof can be made of or include a radiopaque or imaging material.

In the embodiment shown in Figure 8, the coil 870 has a first portion 872 having a first pitch, a second portion 874 having a second pitch greater than the first pitch, and a third portion 876 have a third pitch less than the second pitch. The first and third pitches can be the same or different. The third portion 876 can be distal of the second portion 874, and the second portion 874 can be distal of the first portion 872.

### III. Selected Methods of Manufacture (not claimed)

The outer polymer structure 116 can be constructed and disposed using any appropriate technique, for example, by extrusion, co-extrusion, ILC, coating, heat shrink techniques, heat bonding, casting, molding, fusing one or several segments of an outer polymer structure material end-to-end, or the like. The outer polymer structure 116 can be secured to the inner polymer structure 114, the coil 170, the inner braid 160, and/or the outer braid 162 by any of the above techniques. In embodiments where the outer polymer structure 116 is constructed independently of the other portions of the shaft 106, the outer polymer structure 116 may be thereafter secured to the inner polymer structure 114, the inner braid 160, the outer braid 162, and/or the coil 170 using suitable techniques such as adhesive bonding, crimping, friction fitting, mechanically fitting, chemically bonding, thermally bonding, welding (e.g., resistance, RF, or laser welding), soldering, brazing, or the use of a connector member or material, or the like, or combinations thereof.

### V. Conclusion

Several other embodiments of the technology can have different states, components, or procedures than those described herein. Moreover, it will be appreciated that specific elements, substructures, advantages, uses, and/or other features of the embodiments described with reference to Figures 1A-8 can be suitably interchanged, substituted or otherwise configured with one another in accordance with additional embodiments of the present technology. For example, any of the distal portions described with reference to Figures 3-8 can be combined with any of the elongated shafts and/or catheter systems described with references to Figures 1A-2. Furthermore, suitable elements of the embodiments described with reference to Figures 1A-8 can be used as standalone and/or self-contained devices. A person of ordinary skill in the art, therefore, will accordingly understand that the technology can have other embodiments with additional elements, or the technology can have other embodiments without several of the features shown and described above with reference to Figures 1A-8.

## Claims

1. A catheter, comprising:
an elongated shaft (106, 206) having a proximal portion (106a) and a distal portion (106b, 300, 400, 500, 600, 700, 800), wherein the elongated shaft comprises an inner polymer structure (114, 314, 414, 514, 614, 714, 814) and an outer polymer structure (116, 316, 416, 516, 616, 716, 816) disposed around the inner polymer structure;
a first braid (160, 162) positioned around at least a portion of the inner polymer structure and extending along at least a portion of the shaft;
a coil (170, 370, 470, 570, 670, 770, 870) wound around at least a portion of the inner polymer structure and extending along at least a portion of the shaft, wherein at least a portion of one of the first braid or the coil overlaps at least a portion of the other of the first braid or coil; and
a second braid (160, 162) extending along at least a portion of the shaft,
wherein the outer polymer structure is disposed in and around the first braid and the coil;,
wherein the outer polymer structure has a stiffness that decreases in a distal direction,
wherein the first braid extends distally from the proximal portion of the shaft to a first position along the shaft,
wherein the second braid extends distally from the proximal portion of the shaft to a second position along the shaft that is proximal of the first position,
wherein the coil extends distally from a first location to a second location,
wherein the first location is proximal to the first position, and
wherein the second location is distal to the first position.

2. The catheter of claim 1 wherein the second location is within the distal portion of the shaft.

3. The catheter of claim 1 or 2 wherein at least a portion of the first braid overlaps at least a portion of the coil.

4. The catheter of claim 1 or 2 wherein at least a portion of the coil overlaps at least a portion of the first braid.

5. The catheter of any one of claims 1 to 4, wherein at least a portion of the second braid is positioned around at least a portion of the first braid.

6. The catheter of any one of claims 1 to 3, wherein
at least a portion of the second braid is positioned around at least a portion of the first braid; and
at least a portion of the first braid is positioned around at least a portion of the coil and within the outer polymer structure.

7. The catheter of claim 1, 2 or 4, wherein
at least a portion of the second braid is positioned around at least a portion of the first braid; and
at least a portion of the coil is positioned around at least a portion of the first braid and within the outer polymer structure.

8. The catheter of any one of claims 1 to 7 wherein the outer polymer structure includes a first portion (120, 130, 140) and a second portion (130, 140, 150) distal to the first portion along the length of the outer polymer structure, wherein the first portion has a first stiffness that is constant along the length of the first portion and the second portion has a second stiffness that is constant along the length of the second portion, and wherein the second stiffness is less than the first stiffness.

9. The catheter of any one of claims 1 to 8, further comprising a liner that coats an inner surface of the inner polymer structure.

10. The catheter of any one of claims 1 to 9, further comprising a radiopaque marker (117, 317, 417, 517, 617, 717, 817) positioned along the distal portion.

11. The catheter of any one of claims 1 to 10 wherein the coil has a pitch, and wherein the pitch varies along the length of the coil.

12. The catheter of any one of claims 1 to 11 wherein the first braid has a pitch, and wherein the pitch varies along the length of the first braid.

13. The catheter of claim 1, further comprising:
an intermediate portion (180) between the proximal portion and the distal portion, wherein the distal portion terminates distally at an opening;
wherein the intermediate portion has a first region, a second region, and a third region, wherein
the first region extends from the proximal portion of the shaft to the second region, wherein the first region includes the inner polymer structure, the outer polymer structure, the first braid, and the second, wherein the first braid and the second braid are positioned within the outer polymer structure;
the second region extends from the first region to the third region,
wherein the second region includes the inner polymer structure, the outer polymer structure, a coil wound around the inner polymer structure and positioned within the outer polymer structure, and at least one of the first braid and/or the second braid;
the third region extends from the second region to the distal portion, wherein the third region includes the inner polymer structure, the outer polymer structure, and the coil wound around at least a portion of the inner polymer structure.

14. The catheter of claim 13 wherein a stiffness of the inner polymer structure decreases in a distal direction.

15. The catheter of claim 13 or 14 wherein a stiffness of the outer polymer structure decreases in a distal direction.

16. The catheter of any one of claims 13 to 15 wherein an outer diameter of the shaft at the third region is less than an outer diameter of the shaft at the second region.

17. The catheter of any one of claims 13 to 16 wherein the outer polymer structure is composed of at least two different polymers.

18. The catheter of claim 13 wherein the second region is an overlapping region (180) that extends from a proximal terminus (170a) of the coil to the distal terminus of the outer braid (162b).

## Patentansprüche

1. Katheter, umfassend:
einen länglichen Schaft (106, 206) mit einem proximalen Abschnitt (106a) und einem distalen Abschnitt (106b, 300, 400, 500, 600, 700, 800), wobei der längliche Schaft eine innere Polymerstruktur (114, 314, 414, 514, 614, 714, 814) und eine äußere Polymerstruktur (116, 316, 416, 516, 616, 716, 816), die um die innere Polymerstruktur herum angeordnet ist, umfasst;
ein erstes Geflecht (160, 162), das um mindestens einen Abschnitt der inneren Polymerstruktur herum positioniert ist und sich entlang mindestens einem Abschnitt des Schafts erstreckt;
eine Spule (170, 370, 470, 570, 670, 770, 870), die um mindestens einen Abschnitt der inneren Polymerstruktur gewickelt ist und sich entlang mindestens einem Abschnitt des Schafts erstreckt, wobei mindestens ein Abschnitt eines des ersten Geflechts oder der Spule mindestens einen Abschnitt des anderen des ersten Geflechts oder der Spule überlappt; und
ein zweites Geflecht (160, 162), das sich entlang mindestens einem Abschnitt des Schafts erstreckt,
wobei die äußere Polymerstruktur in und um das erste Geflecht und die Spule herum angeordnet ist,
wobei die äußere Polymerstruktur eine Steifigkeit aufweist, die in distaler Richtung abnimmt,
wobei sich das erste Geflecht distal von dem proximalen Abschnitt des Schafts zu einer ersten Position entlang dem Schaft erstreckt,
wobei sich das zweite Geflecht distal von dem proximalen Abschnitt des Schafts zu einer zweiten Position entlang dem Schaft erstreckt, die proximal zu der ersten Position ist,
wobei sich die Spule distal von einer ersten Stelle zu einer zweiten Stelle erstreckt,
wobei die erste Stelle proximal zu der ersten Position ist und
wobei die zweite Stelle distal zu der ersten Position ist.

2. Katheter nach Anspruch 1, wobei sich die zweite Stelle innerhalb des distalen Abschnitts des Schafts befindet.

3. Katheter nach Anspruch 1 oder 2, wobei mindestens ein Abschnitt des ersten Geflechts mindestens einen Abschnitt der Spule überlappt.

4. Katheter nach Anspruch 1 oder 2, wobei mindestens ein Abschnitt der Spule mindestens einen Abschnitt des ersten Geflechts überlappt.

5. Katheter nach einem der Ansprüche 1 bis 4, wobei mindestens ein Abschnitt des zweiten Geflechts um mindestens einen Abschnitt des ersten Geflechts herum positioniert ist.

6. Katheter nach einem der Ansprüche 1 bis 3, wobei
mindestens ein Abschnitt des zweiten Geflechts um mindestens einen Abschnitt des ersten Geflechts herum positioniert ist und
mindestens ein Abschnitt des ersten Geflechts um mindestens einen Abschnitt der Spule herum und innerhalb der äußeren Polymerstruktur positioniert ist.

7. Katheter nach Anspruch 1, 2, oder 4, wobei
mindestens ein Abschnitt des zweiten Geflechts um mindestens einen Abschnitt des ersten Geflechts herum positioniert ist und
mindestens ein Abschnitt der Spule um mindestens einen Abschnitt des ersten Geflechts herum und innerhalb der äußeren Polymerstruktur positioniert ist.

8. Katheter nach einem der Ansprüche 1 bis 7, wobei die äußere Polymerstruktur einen ersten Abschnitt (120, 130, 140) und einen zweiten Abschnitt (130, 140, 150) distal zum ersten Abschnitt entlang der Länge der äußeren Polymerstruktur einschließt, wobei der erste Abschnitt eine erste Steifigkeit aufweist, die entlang der Länge des ersten Abschnitts konstant ist, und der zweite Abschnitt eine zweite Steifigkeit aufweist, die entlang der Länge des zweiten Abschnitts konstant ist, und wobei die zweite Steifigkeit geringer als die erste Steifigkeit ist.

9. Katheter nach einem der Ansprüche 1 bis 8, ferner umfassend eine Auskleidung, die eine Innenoberfläche der inneren Polymerstruktur beschichtet.

10. Katheter nach einem der Ansprüche 1 bis 9, ferner umfassend einen röntgendichten Markierer (117, 317, 417, 517, 617, 717, 817), der entlang dem distalen Abschnitt positioniert ist.

11. Katheter nach einem der Ansprüche 1 bis 10, wobei die Spule eine Steigung aufweist und wobei die Steigung entlang der Länge der Spule variiert.

12. Katheter nach einem der Ansprüche 1 bis 11, wobei das erste Geflecht eine Steigung aufweist und wobei die Steigung entlang der Länge des ersten Geflechts variiert.

13. Katheter nach Anspruch 1, ferner umfassend:
einen Zwischenabschnitt (180) zwischen dem proximalen Abschnitt und dem distalen Abschnitt, wobei der distale Abschnitt distal an einer Öffnung endet,
wobei der Zwischenabschnitt einen ersten Bereich, einen zweiten Bereich und einen dritten Bereich aufweist, wobei
der erste Bereich sich von dem proximalen Abschnitt des Schafts zu dem zweiten Bereich erstreckt, wobei der erste Bereich die innere Polymerstruktur, die äußere Polymerstruktur, das erste Geflecht und das zweite einschließt, wobei das erste Geflecht und das zweite Geflecht innerhalb der äußeren Polymerstruktur positioniert sind;
der zweite Bereich sich von dem ersten Bereich zu dem dritten Bereich erstreckt, wobei der zweite Bereich die innere Polymerstruktur, die äußere Polymerstruktur, eine Spule, die um die innere Polymerstruktur gewickelt ist und innerhalb der äußeren Polymerstruktur positioniert ist, und mindestens eines von dem ersten Geflecht und/oder dem zweiten Geflecht einschließt;
der dritte Bereich sich von dem zweiten Bereich zu dem distalen Abschnitt erstreckt, wobei der dritte Bereich die innere Polymerstruktur, die äußere Polymerstruktur und die Spule, die um mindestens einen Abschnitt der inneren Polymerstruktur gewickelt ist, einschließt.

14. Katheter nach Anspruch 13, wobei eine Steifigkeit der inneren Polymerstruktur in einer distalen Richtung abnimmt.

15. Katheter nach Anspruch 13 oder 14, wobei eine Steifigkeit der äußeren Polymerstruktur in einer distalen Richtung abnimmt.

16. Katheter nach einem der Ansprüche 13 bis 15, wobei ein Außendurchmesser des Schafts am dritten Bereich geringer als ein Außendurchmesser des Schafts am zweiten Bereich ist.

17. Katheter nach einem der Ansprüche 13 bis 16, wobei die äußere Polymerstruktur aus mindestens zwei unterschiedlichen Polymeren besteht.

18. Katheter nach Anspruch 13, wobei der zweite Bereich ein Überlappungsbereich (180) ist, der sich von einem proximalen Ende (170a) der Spule zu dem distalen Ende des äußeren Geflechts (162b) erstreckt.

## Revendications

1. Cathéter comprenant :
un arbre allongé (106, 206) ayant une partie proximale (106a) et une partie distale (106b, 300, 400, 500, 600, 700, 800), dans lequel l'arbre allongé comprend une structure polymère interne (114, 314, 414, 514, 614, 714, 814) et une structure polymère externe (116, 316, 416, 516, 616, 716, 816) disposée autour de la structure polymère interne ;
une première tresse (160, 162) positionnée autour d'au moins une partie de la structure polymère interne et s'étendant le long d'au moins une partie de l'arbre ;
une bobine (170, 370, 470, 570, 670, 770, 870) enroulée autour d'au moins une partie de la structure polymère interne et s'étendant le long d'au moins une partie de l'arbre, dans lequel au moins une partie de l'une parmi la première tresse ou la bobine chevauche au moins une partie de l'autre parmi la première tresse ou bobine ; et
une seconde tresse (160, 162) s'étendant le long d'au moins une partie de l'arbre,
dans lequel la structure polymère externe est disposée dans et autour de la première tresse et de la bobine,
dans lequel la structure polymère externe a une rigidité qui diminue dans une direction distale,
dans lequel la première tresse s'étend de manière distale depuis la partie proximale de l'arbre jusqu'à une première position le long de l'arbre,
dans lequel la seconde tresse s'étend de manière distale depuis la partie proximale de l'arbre jusqu'à une seconde position le long de l'arbre qui est proximale par rapport à la première position,
dans lequel la bobine s'étend de manière distale d'un premier emplacement à un second emplacement,
dans lequel le premier emplacement est proximal à la première position, et
dans lequel le second emplacement est distal par rapport à la première position.

2. Cathéter selon la revendication 1, dans lequel le second emplacement est à l'intérieur de la partie distale de l'arbre.

3. Cathéter selon la revendication 1 ou 2, dans lequel au moins une partie de la première tresse chevauche au moins une partie de la bobine.

4. Cathéter selon la revendication 1 ou 2, dans lequel au moins une partie de la bobine chevauche au moins une partie de la première tresse.

5. Cathéter selon l'une quelconque des revendications 1 à 4, dans lequel au moins une partie de la seconde tresse est positionnée autour d'au moins une partie de la première tresse.

6. Cathéter selon l'une quelconque des revendications 1 à 3, dans lequel
au moins une partie de la seconde tresse est positionnée autour d'au moins une partie de la première tresse ; et
au moins une partie de la première tresse est positionnée autour d'au moins une partie de la bobine et à l'intérieur de la structure polymère externe.

7. Cathéter selon la revendication 1, 2 ou 4, dans lequel
au moins une partie de la seconde tresse est positionnée autour d'au moins une partie de la première tresse ; et
au moins une partie de la bobine est positionnée autour d'au moins une partie de la première tresse et à l'intérieur de la structure polymère externe.

8. Cathéter selon l'une quelconque des revendications 1 à 7, dans lequel la structure polymère externe comporte une première partie (120, 130, 140) et une seconde partie (130, 140, 150) distale par rapport à la première partie le long de la longueur de la structure polymère externe, dans lequel la première partie a une première rigidité qui est constante le long de la longueur de la première partie et la seconde partie a une seconde rigidité qui est constante le long de la longueur de la seconde partie, et dans lequel la seconde rigidité est inférieure à la première rigidité.

9. Cathéter selon l'une quelconque des revendications 1 à 8, comprenant en outre un revêtement qui recouvre une surface interne de la structure polymère interne.

10. Cathéter selon l'une quelconque des revendications 1 à 9, comprenant en outre un marqueur radio-opaque (117, 317, 417, 517, 617, 717, 817) positionné le long de la partie distale.

11. Cathéter selon l'une quelconque des revendications 1 à 10, dans lequel la bobine a un pas, et dans lequel le pas varie le long de la longueur de la bobine.

12. Cathéter selon l'une quelconque des revendications 1 à 11, dans lequel la première tresse a un pas, et dans lequel le pas varie le long de la longueur de la première tresse.

13. Système selon la revendication 1, comprenant en outre :
une partie intermédiaire (180) entre la partie proximale et la partie distale, dans lequel la partie distale se termine de manière distale au niveau d'une ouverture ;
dans lequel la partie intermédiaire a une première région, une deuxième région et une troisième région, dans lequel
la première région s'étend depuis la partie proximale de l'arbre jusqu'à la deuxième région, dans lequel la première région comporte la structure polymère interne, la structure polymère externe, la première tresse, et la seconde, dans lequel la première tresse et la seconde tresse sont positionnées à l'intérieur de la structure polymère externe ;
la deuxième région s'étend depuis la première région jusqu'à la troisième région, dans lequel la deuxième région comporte la structure polymère interne, la structure polymère externe, une bobine enroulée autour de la structure polymère interne et positionnée à l'intérieur de la structure polymère externe, et au moins l'une parmi la première tresse et/ou la seconde tresse ;
la troisième région s'étend depuis la deuxième région jusqu'à la partie distale, dans lequel la troisième région comporte la structure polymère interne, la structure polymère externe et la bobine enroulée autour d'au moins une partie de la structure polymère interne.

14. Cathéter selon la revendication 13, dans lequel une rigidité de la structure polymère interne diminue dans une direction distale.

15. Cathéter selon la revendication 13 ou 14, dans lequel une rigidité de la structure polymère externe diminue dans une direction distale.

16. Cathéter selon l'une quelconque des revendications 13 à 15, dans lequel un diamètre externe de l'arbre au niveau de la troisième région est inférieur à un diamètre externe de l'arbre au niveau de la deuxième région.

17. Cathéter selon l'une quelconque des revendications 13 à 16, dans lequel la structure polymère externe est composée d'au moins deux polymères différents.

18. Cathéter selon la revendication 13, dans lequel la deuxième région est une région de chevauchement (180) qui s'étend d'une extrémité proximale (170a) de la bobine à l'extrémité distale de la tresse externe (162b).
